# EUROPEAN PATENT APPLICATION

(11) **EP 4 001 422 A1**
(43) Date of publication of application: **25.05.2022**
(21) Application number: 20208900.9
(22) Date of filing: 20.11.2020
(51) Int. Cl.: C12P 13/00

(54) **PREPARATION OF OPTICALLY ACTIVE CYANOHYDRINS FROM ALDEHYDES AND KETONES USING A BIPHASIC HYDROXYNITRILE LYASE-CATALYZED HYDROCYANATION PROCESS**

(71) Applicant: Enzymaster Deutschland GmbH, 40219 Düsseldorf (DE)
(72) Inventor: VAN LANGEN, Luuk, 2611CZ Delft (NL); CHEN, Haibin, Zhejiang, Ningbo 315000 (CN); CAI, Baoqin, Zhejiang, Ningbo 315000 (CN); DAUSSMAN, Thomas, 25889 Witzwort (DE)
(74) Representative: Lederer & Keller Patentanwälte Partnerschaft mbB

(57) **Abstract**

Use of an aqueous salt solution having a concentration of at least 0.8 mol/L for improving the enantioselectivity in a process for the preparation of optically active cyanohydrins from aldehydes and ketones with hydrogen cyanide in the presence of hydroxynitrile lyase in a biphasic solvent system further comprising an organic solvent.

## Description

### Technical Field

The present invention relates to the use of a highly concentrated aqueous salt solution in a biphasic hydroxynitrile lyase-catalyzed hydrocyanation process for the preparation of optically active cyanohydrins from aldehydes and ketones with improved enantiomeric excess.

### Background

Enantiopure cyanohydrins are versatile synthetic building blocks. Their synthesis via hydroxynitrile lyase-catalyzed hydrocyanation of aldehydes or ketones has been studied intensively, including the development of industrially viable processes.

The process is typically performed in a biphasic system including a water-immiscible solvent and an aqueous buffer. The reactants are accumulated in the organic phase and the hydrocyanation then takes place in the aqueous phase which comprises the enzyme.

In this two-phase system concept, the enantiopurity of the product (indicated by the enantiomeric excess (abbreviated as "ee")) is determined by the intrinsic (enantio)selectivity of the enzyme minus the contribution of the blank reaction, i.e. the non-catalyzed spontaneous chemical reaction between the aldehyde or ketone and the cyanide source, which leads to the formation of racemic cyanohydrin. The rates of both processes depend on the structure of the reactant and the reaction conditions.

US 5,350,871, for example, discloses a method of preparing optically active cyanohydrins by addition of hydrogen cyanide to a carbonyl compound selected from aldehydes and ketones in the presence of hydroxynitrile lyase in a biphasic solvent system, comprising a homogenous aqueous solution of the enzyme and a suitable organic solvent which is substantially immiscible with water. The method is performed in that the homogenous aqueous solution is buffered with an acetate buffer or with a non-acetate buffer such as a citrate, succinate, glutamate or phthalate buffer.

The method disclosed in US 5,350,871 allows the provision of optically active cyanohydrins with enantiomeric excesses up to 98%. On the other hand, US 5,350,871 discloses that 4-hydroxybenzaldehyde represents a notoriously difficult substrate for the preparation of the corresponding optically active cyanohydrin. Indeed, 4-hydroxymandelonitrile was obtained with only 94% ee in US 5,350,871.

US 2002/0052523 A1, wherein the enzymatic hydrocyanation is performed in an emulsion derived from a biphasic system by rapidly stirring the reaction mixture, also discloses that 4-hydroxybenzaldehyde and 3,4-dihydroxybenzaldehyde have reduced reactivity in the hydroxynitrile lyase-catalyzed hydrocyanation.

Different strategies have been applied to improve the relative rate of the enzymatic, enantioselective hydrocyanation versus the chemical, racemic hydrocyanation, especially for aldehydes, where the ratio of enzymatic hydrocyanation versus chemical hydrocyanation is low, as it is the case for 4-hydroxybenzaldehyde compared to the well-performing aldehyde benzaldehyde as shown in US 5,350,871.

Such strategies can essentially be classified into two approaches. The first approach aims at the improvement of the selectivity rate of the enzyme for a certain aldehyde. The second approach aims at the adjustment of the reaction conditions for improving the ratio of enzymatic hydrocyanation versus chemical hydrocyanation.

An example for the first approach is disclosed in US 2010/0143986 A1, wherein a recombinant variant of (R)-hydroxynitrile lyase was used for preparing enantiomerically pure cyanohydrins from sterically hindered aldehydes, in particular from 2-chlorobenzaldehyde and hydroxypivaldehyde.

The second approach uses the adaption of reaction parameters, including e.g. the dose of the enzyme, the temperature, the pH, the ratio of organic phase to aqueous phase etc., in order to increase the ratio of enzymatic, enantioselective hydrocyanation versus chemical, racemic hydrocyanation.

In the afore-mentioned US 2010/143986 A1, the influence of the buffer concentration on the enantiomeric excess of the cyanohydrin resulting from hydroxypivaldehyde was investigated in a monophasic aqueous system. While buffer concentrations of 1M and more have been compared to lower buffer concentrations, it was found that the enantiomeric excess remained roughly constant throughout the concentrations tested.

US 2004/0048346 A1 discloses a process for preparing (R)-2-hydroxy-4-phenylbutyronitrile which comprises reacting in a biphasic system 3-phenylpropionaldehyde with a cyanide compound in the presence of (R)-hydroxynitrile lyase, wherein the reaction is carried out at a temperature below 10°C, providing the target cyanohydrin with an enantiomeric excess of 98%. The aqueous phase preferably comprises a buffering agent such as an acetate buffer, or a non-acetate buffer, e.g. citrate, glutamate, succinate, or phthalate buffer. While the document discloses a buffer concentration of up to 1M, the document favors buffer concentrations below 1M in order to avoid problems with the phase separation and subsequent workup of the reaction mixture.

Willeman, WF et al. (Biotech. Bioeng. 79(2), 2002, 154-164) have studied the kinetic parameters of the hydrocyanation of 4-hydroxybenzaldehyde by purified almond (R)-hydroxynitrile lyase and found that the equilibrium constant for 4-hydroxymandelonitrile formation is not as favorable as for mandelonitrile. Also, the enzymatic rate of 4-hydroxymandelontrile formation was much lower than for mandelonitrile, while the blank reaction to 4-hydroxymandelontrile formation was much higher than for mandelonitrile. Using high amounts of enzyme and optimized volume of aqueous phase (pH 5.5), and low temperature (1-3°C) provided (*R*)-4-hydroxymandelonitrile with 90% conversion and with 95% ee in 20 h.

It is the object of the present invention to provide a means and method for improving the enantiomeric excess in a process for the preparation of optically active cyanohydrins from aldehydes and ketones with hydrogen cyanide in the presence of hydroxynitrile lyase which is carried out a biphasic system.

### Summary of the invention

This object is solved by the subject matter in any one of the following items:
1. Use of an aqueous salt solution having a concentration of at least 0.8 mol/L for improving the enantioselectivity in a process for the preparation of optically active cyanohydrins from aldehydes and ketones with hydrogen cyanide in the presence of hydroxynitrile lyase in a biphasic solvent system further comprising an organic solvent.
2. Use according to item 1, wherein the aqueous salt solution comprises a buffer salt solution or a mixture of a buffer salt solution and a non-buffer salt solution.
3. Use according to item 2, wherein the buffer is a glutamate, phosphate, acetate or citrate buffer.
4. Use according to any one of the preceding items, wherein the aqueous salt solution comprises a buffer and an alkali or earth alkali salt, preferably an alkali or earth alkali sulfate.
5. Use according to any one of the preceding items, wherein the aqueous salt solution comprises a mixture of a citrate buffer and sodium sulfate.
6. Use according to any one pf the preceding items, wherein the concentration of the aqueous salt solution is at least 1 mol/L, preferably at least 1.2 mol/L, more preferably at least 1.4 mol/L.
7. Use according to any one of the preceding items, wherein hydroxynitrile lyase is used in an amount in the range from 1 to 1000 U per mmol of the aldehyde or ketone.
8. Use according to any one of the preceding items, wherein the pH of the aqueous salt solution is in the range from 3 to 7.
9. Use according to any one of the preceding items, wherein the organic solvent is an aliphatic ether or a mixture of aliphatic ethers, preferably di-*iso*-propylether and methyl-*tert-*butylether.
10. Use according to any one of the preceding items, wherein the process is carried out at a temperature ranging from 0°C to 30°C.
11. Use according to any one of preceding items, wherein the aldehydes and ketones are compounds of the following formula (I) 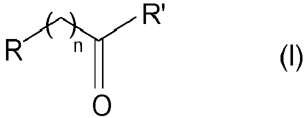 wherein in formula (I)
   n = 0, 1 or 2,
   R = aryl, heteroaryl or alkyl
   R' = H or C1-C4 alkyl, whe
      rein
      R = aryl means a moiety of the following formula (II) 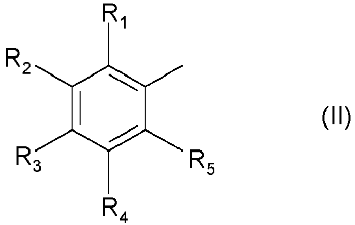 wherein
         R₁-R₅ are independently selected from -H, -F, -Cl, -Br, -I, -OH, -O(C1-C4 alkyl), -O-benzyl, -OCO(C1-C4 alkyl), -OCO-benzyl, -NH₂, - NH(C1-C4 alkyl), -NH-benzyl, -NHCO(C1-C4 alkyl), -N-HCO-benzyl, - NO₂, -COOH, -COO(C1-C4 alkyl), -COO-benzyl, wherein preferably at least one of R₁-R₅ is -OH;
      R = heteroaryl means a moiety selected from the group consisting of 2-pyrrolyl, 2-furyl, 2-thienyl, 3-thienyl, 2-pyridyl, 3-pyridyl, 3-pyrrolyl, 3-furyl;
      R = alkyl means C1-C4 alkyl, optionally substituted with -OH, -O(C1-C4 alkyl), -O-benzyl, -OCO(C1-C4 alkyl), -OCO-benzyl, -NH₂, -NH(C1-C4 alkyl), -NH-benzyl, -NHCO(C1-C4 alkyl), -N-HCO-benzyl, -NO₂, -COOH, -COO(C1-C4 alkyl), -COO-benzyl.
12. Use according to item 11, wherein in the compound of the formula (I) n = 0, 1 or 2, R = aryl or heteroaryl, and R' = H or C1-C4 alkyl.
13. Use according to item 11 or 12, wherein in the compound of the formula (I) n = 0, R = aryl or heteroaryl, and R' = H
14. Use according to any one of items 11 to 13, wherein the compound of formula (I) is selected from hydroxy- and di-hydroxy-substituted benzaldehyde analogues.
15. Use according to any of the preceding items, wherein the hydroxynitrile lyase is immobilized hydroxynitrile lyase.

Further details and embodiments of the present invention are described in the following detailed description of the invention.

### Detailed description

The present invention is based on the surprising finding of the inventors that it is advantageous to use a highly concentrated aqueous salt solution with a salt concentration of at least 0.8 mol/L, preferably at least 1 mol/L, in an enzymatically catalyzed hydrocyanation process of aldehydes and ketones with hydrogen cyanide in the presence of hydroxynitrile lyase ((*R*) or (*S*) HNL), wherein the process is carried out in a biphasic solvent system comprising said aqueous salt solution and an organic solvent, in order to obtain optically active cyanohydrins with improved enantiomeric excess.

"Improved enantiomeric excess" in the context of the present invention means that the enantiomeric excess of the cyanohydrin resulting from a process as described herein for the preparation of optically active cyanohydrins is higher compared to a cyanohydrin prepared in a comparative process, which in the ideal case is carried out under similar reaction conditions (i.e. using same reactants and enzyme in the same amounts, the same solvents in the same volumes, the same temperature, and the same pH), and only differs from the process used in the present invention in that the aqueous salt solution has a concentration that is at least 10 times, preferably 20 times lower than the concentration of the aqueous salt solution used in the process of the present invention. If for example in the process of the present invention the aqueous salt solution has a concentration of 1 mol/L, then the comparative process uses an aqueous salt solution at a concentration of 0.1 mol/L, preferably 0.05 mol/L.

If for example 4-hydroxybenzaldehyde, which represents a particularly problematic substrate with respect to achieving high enantiomeric excess as confirmed in the prior art (US 5,350,871, Example VIII, and US 2002/0052523 A1, paragraph [0022]), is hydrocyanated in a process used in the present invention using a highly concentrated salt solution having a concentration of at least 0.8 mol/L, preferably at least 1 mol/L, it has been found that the resulting optically active cyanohydrin, 4-hydroxymandelonitrile, can be prepared with an enantiomeric excess of not below 99% when 76% of the 4-hydroxybenzaldehyde has been converted, and an enantiomeric excess not below 98% when 81% of the 4-hydroxybenzaldehyde has been converted.

The enantiomeric excess of the optically active cyanohydrins resulting from the process used in the present invention can be determined with common techniques, such as chiral HPLC.

The aqueous salt solution used in the present invention comprises a mixture of ions, including buffering and/or non-buffering ions.

In a preferred embodiment, the aqueous salt solution comprises a buffer salt solution (also referred to herein as a "buffer").

The term "buffer" or "buffer salt solution" as used in the present invention is to be understood in accordance with the common understanding of the skilled person. Accordingly, the term "buffer" or "buffer salt solution" refers to a pH buffer in aqueous solution, which comprises a mixture of an acid and its conjugate base in order to keep the pH at a nearly constant value. The conjugate base is used in form of a salt, typically an alkali or earth alkali salt, such as sodium, potassium, magnesium and calcium salts. Acid component and conjugate base component are typically used in equimolar amounts in the aqueous buffer. The pH of an aqueous buffer solution can also be adjusted by means of the ratio of acid and conjugate base used in the aqueous solution.

If a buffer salt solution (i.e. a buffer) is used as the aqueous salt solution in the present invention, the concentration of the aqueous salt solution means the concentration of the conjugate base of the buffer, i.e. the concentration of the buffer salt.

Suitable buffers for use in the present invention include buffers commonly known to the skilled person for use in the catalytic hydrocyanation of aldehydes and ketones (as e.g. used in US 2002/0052523 A1 and US 2004/0048346 A1). Preferably, the buffers for use in the present invention are selected from the group consisting of glutamate buffers (e.g. glutamic acid-glutamate), phosphate buffers (e.g. phosphoric acid-phosphate), acetate buffers (e.g. acetic acid-acetate), and citrate buffers (e.g. citric acid-citrate).

Preferably, a citric acid-citrate buffer is used in the present invention, with the citrate being used in form of an alkali metal salt, such as e.g. sodium or potassium citrate. The potassium citrate buffer is particularly preferred.

The pH of the buffer depends on the pkₐ value of the acid component and the concentrations of acid and conjugate base component used in the buffer. Which buffer is to be used for a certain pH (range) forms part of the common general knowledge of the skilled person.

Mixtures if different buffers can also be used in the present invention. In that case, the concentration of the aqueous salt solution means the sum of the buffer salt concentrations.

In a further preferred embodiment, the aqueous salt solution comprises a non-buffer salt.

Non-buffer salts for use in the present invention include alkali or earth alkali metal salts, in particular their halides and sulfates such as e.g. NaCl, NaBr, Nal, Na₂SO₄, KCl, KBr, KI, K₂SO₄, MgCl₂, MgBr₂, Mgl₂, MgSO₄, CaCl₂, CaBr₂, Cal₂, and CaSO₄. Alkali and earth alkali metal sulfates are preferably used in the present invention, and of these, the use of alkali sulfates is more preferred, with sodium sulfate being particularly preferred.

Mixtures of different non-buffer salts can also be used in the present invention. In that case, the concentration of the aqueous salt solution means the sum of non-buffer salt concentrations.

In a further preferred embodiment of the present invention, the aqueous salt solution comprises a mixture of a buffer salt solution and a non-buffer salt solution. In that case, the concentration of the aqueous salt solution means the sum of the non-buffer salt concentration and of the buffer salt concentration. For example, a mixture of a buffer at a buffer salt concentration of 0.05 mol/L and a non-buffer salt at a concentration of 0.95 mol/L adds up to a concentration of the aqueous salt solution of 1 mol/L.

Concentration ratios of buffer salt and non-buffer salt in the aqueous salt solution typically used in the present invention can range from 1:100 to 100:1. For example, at a concentration of the aqueous salt solution of 1 mol/L, the following ratios are included: buffer 0.01 mol/L, non-buffer salt 0.99 mol/L; buffer 0.02 mol/L, non-buffer salt 0.98 mol/L; buffer 0.05 mol/L, non-buffer salt 0.95 mol/L; buffer 0.1 mol/L, non-buffer salt 0.9 mol/L; buffer 0.2 mol/L, non-buffer salt 0.8 mol/L; buffer 0.4 mol/L, non-buffer salt 0.6 mol/L; buffer 0.5 mol/L, non-buffer salt 0.5 mol/L; buffer 0.6 mol/L, non-buffer salt 0.4 mol/L; buffer 0.8 mol/L, non-buffer salt 0.2 mol/L; buffer 0.9 mol/L, non-buffer salt 0.1 mol/L; buffer 0.95 mol/L, non-buffer salt 0.05 mol/L; buffer 0.98 mol/L, non-buffer salt 0.02 mol/L; buffer 0.99 mol/L, non-buffer salt 0.01 mol/L. Analogous ratios can be defined if the concentration of the aqueous salt solution comprising a buffer and non-buffer salt is higher than 1 mol/L, such as e.g. 1.2 mol/L, 1.4 mol/L, etc..

In a further preferred embodiment of the present invention, the aqueous salt solution comprises a buffer and an alkali sulfate. The buffer is preferably a citrate buffer, more preferably a potassium citrate buffer, and the alkali sulfate is preferably sodium sulfate. In an aqueous salt solution comprising a citrate buffer and sodium sulfate, the citrate buffer is preferably used at a concentration of 0.05 mol/L and the sodium sulfate preferably at a concentration of 0.95 mol/L.

While a concentration of the aqueous salt solution of at least 0.8 mol/L, preferably at least 1 mol/L, more preferably at least 1.2 mol/L, even more preferably at least 1.4 mol/L, at least 1.5 mol/L, even more preferably at least 1.7 mol/L, even more preferably 1.9 mol/L, even more preferably 2.0 mol/L, even more preferably at least 2.5 mol/L, even more preferably at least 3 mol/L is required according to the present invention for preparing optically active cyanohydrins with improved enantiomeric excess, it is preferred that the concentration of the aqueous salt solution is below the saturation concentration of the salt in water in order to ensure that the aqueous phase is still a solution, which is important in order that the reaction mixture can smoothly been stirred. Preferably, the concentration of the aqueous salt solution is not higher than 10 mol/L, more preferably not higher than 9 mol/L, even more preferably not higher than 8 mol/L, even more preferably not higher than 7 mol/L, even more preferably not higher than 6 mol/L, even more preferably not higher than 5 mol/L, even more preferably not higher than 4 mol/L.

A low pH of the aqueous salt solution is in favor of the enantioselectivity of the reaction. On the other hand, most hydroxynitrile lyases are less stable at pH values below 3. The process used in the present invention is therefore typically carried out at a pH of the aqueous salt solution in the range from 3 to 7, preferably at a pH in the range from 3.5 to 6.5, even more preferably at a pH in the range from 4 to 6, even more preferably at a pH in the range from 4.5 to 5.5. It is in particular preferred to carry out the process of the present invention at a pH ranging from 4.5 to 5. This can e.g. be achieved using a citric acid- citrate buffer, which is the preferred buffer for use in the process of the present invention.

Suitable organic solvents for use in the present invention are selected from the group consisting of aliphatic ethers, esters of carboxylic acids, aliphatic alcohols, aliphatic ketones, aromatic hydrocarbons, chlorinated aliphatic or aromatic hydrocarbons.

Aliphatic esters include, but are not limited to: diethylether, di-*n*-propylether, di-*iso-*propylether, methyl-*tert*-butylether, di-*n*-butylether, di-*iso*-butylether, or mixtures thereof. Esters of carboxylic acids include, but are not limited to ethyl acetate. Aliphatic alcohols include, but are not limited to: methanol, ethanol, *n*-propanol, *n*-butanol, and *iso*-butanol, or mixtures thereof. Aliphatic ketones include, but are not limited to acetone. Aromatic hydrocarbons include, but are not limited to: toluene, *ortho-, meta-* and/or para-xylene, or mixtures thereof. Chlorinated aliphatic or aromatic hydrocarbons include, but are not limited to: methylene chloride, dichloroethane, trichloroethane, chloroform, chlorobenzene, dichlorobenzene and trichlorobenzene, or mixtures thereof.

While chlorinated aliphatic and aromatic hydrocarbons in principle work in the process of the present invention, they are not particularly preferred due to their toxicity and economic incompatibility. Preferred organic solvents for use in the process of the present invention are aliphatic ethers, in particular di-*iso*-propylether and methyl-*tert*-butylether (MTBE).

The aqueous phase in a biphasic catalytic hydrocyanation process as used in the present invention is required in order for the hydroxynitrile lyase to function, i.e. to catalyze the enantioselective hydrocyanation of aldehydes and ketones. On the other hand, the rate of the racemic blank reaction is also proportional to the volume of the aqueous phase, i.e. a higher amount of water in the reaction mixture would be expected to increase the amount of racemic cyanohydrin formed, thereby decreasing the enantioselectivity of the reaction. In the process of the present invention, the ratio of organic solvent to aqueous salt solution is typically in the range from 8:1 to 4:1, preferably from 7.5:1 to 4.5:1, more preferably from 7:1 to 5:1. Such ratios provide good compromise in terms of the activity of the hydroxynitrile lyase and suppression of the blank reaction that lowers the enantiomeric excess of the reaction.

The concentration of the aldehyde or ketone in the organic solvent is typically from 0.5 to 4 mol/L, preferably from 0.7 to 3.8 mol/L, more preferably from 0.9 to 3.3 mol/L, even more preferably from 1 to 3 mol/L in the process used in the present invention.

Typically, from 1.1 to 5 mmol, preferably from 1.3 to 4.8, more preferably from 1.5 to 4.5 mmol, even more preferably from 1.8 to 4.2 mmol, even more preferably from 2 to 4 mmol hydrogen cyanide are used per mmol of the aldehyde or ketone in the process of the present invention are used, which ensures a high conversion. The concentration of the hydrogen cyanide in the organic solvent is typically from 1 to 5 mol/L, preferably from 1.3 to 4.8 mol/L, more preferably from 1.5 to 4.5 mol/L, even more preferably from 1.8 to 4.3 mol/L, even more preferably from 2 to 4 mol/L in the process used in the present invention.

The process used in the present invention can be carried out at temperature up to 60°C. Preferably, it is carried out at a temperature ranging from 0°C to room temperature (20-30°C), more preferably at lower temperature within this range, such as 10°C, and, particularly preferably, 0°C. A low temperature is advantageous in terms of the enantioselectivity of the reaction, however, the reaction might take longer to reach high conversion.

Aldehydes and ketones preferably used in the present invention include compounds of the following formula (I) 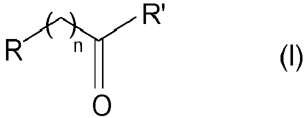 wherein in formula (I)
n = 0, 1 or 2,
R = aryl, heteroaryl or alkyl
R' = H or C1-C4 alkyl,
   wherein
   R = aryl means a moiety of the following formula (II) 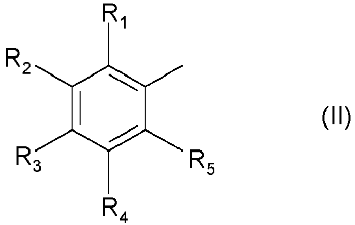 wherein
      R₁-R₅ are independently selected from -H, -F, -Cl, -Br, -I, -OH, -O(C1-C4 alkyl), -O-benzyl, -OCO(C1-C4 alkyl), -OCO-benzyl, -NH₂, - NH(C1-C4 alkyl), -NH-benzyl, -NHCO(C1-C4 alkyl), -N-HCO-benzyl, - NO₂, -COOH, -COO(C1-C4 alkyl), -COO-benzyl, wherein preferably at least one of R₁-R₅ is -OH;
   R = heteroaryl means a moiety selected from the group consisting of 2-pyrrolyl, 3-pyrrolyl, 2-furyl, 3-furyl, 2-thienyl, 3-thienyl, 2-pyridyl, 3-pyridyl;
   R = alkyl means C1-C4 alkyl, optionally substituted -OH, -O(C1-C4 alkyl), -O-benzyl, -OCO(C1-C4 alkyl), -OCO-benzyl, -NH₂, -NH(C1-C4 alkyl), -NH-benzyl, -NHCO(C1-C4 alkyl), -N-HCO-benzyl, -NO₂, -COOH, -COO(C1-C4 alkyl), -COO-benzyl.

If R = alkyl in formula (I), alkyl is preferably selected from selected from methyl, ethyl, *n-*propyl, *iso*-propyl, *n*-butyl, *iso*-butyl, *tert*-butyl, hydroxy-methyl, 2-hydroxy-ethyl, 3-hydroxy-*n*-propyl, 2-hydroxy-*n*-propyl, 4-hydroxy-*n*-butyl, 3-hydroxy-*n*-butyl and 2-hydroxy-*n*-butyl.

If R = heteroaryl in formula (I), heteroaryl is preferably selected from 2-furyl, 3-furyl, 2-pyridyl and 3-pyridyl. More preferably, heteroaryl is selected from 2-furyl and 3-furyl.

If R = aryl in formula (I), R₁-R₅ in the moiety according to formula (II) are preferably independently selected from -H, -F, -Cl, -OH, -OMe, -OCOMe, -NH₂, -NHMe, -NHCOMe, and -NO₂, more preferably from -H, -F, -Cl, -OH, and -NH₂, wherein preferably at least one at least one of R₁-R₅ is -OH. This includes embodiments, wherein one of R₁-R₅, e.g. R₁, R₂, R₃, R₄ or R₅, is -OH, two of R₁-R₅, e.g. R₁ and R₂, R₁ and R₃, R₁ and R₄, R₁ and R₅, R₂ and R₃, R₂ and R₄, are -OH, or three of R₁-R₅ are -OH. In these embodiments, wherein at least one at least one of R₁-R₅ is -OH, the remaining R1-R5 can e.g. be hydrogen.

The less sterically hindered the moiety according to formula (II) is in R₁- and Rs-position, the better is the conversion in the process of the present invention. Therefore, preferably at least one of R₁ and R₅ of the moiety according to formula (II) is hydrogen, preferably both of R₁ and R₅ are hydrogen.

If R' = C1-C4 alkyl in the compound of formula (I), alkyl is methyl, ethyl, *n*-propyl, *iso*-propyl, *n*-butyl or *iso*-butyl.

Preferably, the aldehyde for use in the present invention is not 3-phenylpropionaldehyde.

In a preferred embodiment of the present invention, the compounds according to formula (I) are limited to n = 0, 1 or 2, R = aryl or heteroaryl, and R' = H or C1-C4 alkyl.

In a further preferred embodiment of the present invention, the compounds according to formula (I) are limited to n = 0, R = aryl or heteroaryl, and R' = H or C1-C4 alkyl.

Preferably, aldehydes are used in the present invention, i.e. in compounds according to formula (I) R' = H.

In a more preferred embodiment of the present invention, the compounds according to formula (I) are limited to n = 0, R = aryl or heteroaryl, and R' = H.

In an even more preferred embodiment of the present invention, the compounds according to formula (I) are limited to n = 0, R = aryl, and R' = H.

Particularly preferred compounds for use in the present invention are hydroxy- and di-hydroxy-substituted benzaldehyde analogues, including, but not limited to, hydroxy- and di-hydroxybenzaldehydes, i.e. 2-hydroxybenzaldehyde, 3-hydroxybenzaldehyde, 4-hydroxybenzaldehyde, 2,3-dihydroxybenzaldehyde, 2,4-dihydroxybenzaldehyde, 2,5-dihydroxybenzaldehyde, 2,6-dihydroxybenzaldehyde, 3,4-dihydroxybenzaldehyde, and 3,5-hydroxybenzaldehyde. Preferred examples include 4-hydroxybenzaldehyde and 3,4-dihydroxybenzaldehyde, with 4-hydroxybenzaldehyde being most preferred.

The hydroxynitrile lyase for use in the process of present invention include both (*R*)-hydroxynitrile lyase or (*S*)-hydroxynitrile lyase. Examples for (*S*)-hydroxynitrile lyases include (*S*)-hydroxynitrile lyases from *Manihot esculenta* and *Hevea brasiliensis.* (*R*)-hydroxynitrile lyase is preferably derived from almonds, *Prunus amygdalus sativa* (*P*aHNL).

Typically, at least 1 unit, preferably 10 units, more preferably 50 units, even more preferably 100 units, even more preferably at least 150 units, even more preferably at least 200 units, even more preferably at least 250 units, even more preferably at least 300 units of hydroxynitrile lyase (HNL) are used per mmol aldehyde or ketone in the process of the present invention. Increasing the amount of hydroxynitrile lyase in the reaction system will reduce the relative rate of the blank reaction. In particular, when the process is to be performed in an economical manner, the amount of enzyme used is to be limited. In the process of the present invention, preferably not more than 1000 units, more preferably not more than 950 units, even more preferably not more than 900 units, even more preferably not more than 850 units, even more preferably not more than 800 units, even more preferably not more than 750 units, even more preferably not more than 700 units, even more preferably not more than 650 units, even more preferably not more than 600 units, even more preferably not more than 500 units, even more preferably not more than 400 units of hydroxynitrile lyase are used per mmol aldehyde or ketone.

One "unit" of HNL is defined as the amount of enzyme that results in the formation of 1 µmol benzaldehyde from mandelonitrile per minute. This amount can be determined according to the following activity assay (carried out at 20°C): to 2 mL 50 mM potassium citrate buffer, pH 5, 3 µL R,S-mandelonitrile is added and dissolved by shaking (end concentration: 12.5 mM mandelonitrile). A quartz cuvette is directly filled with 1 mL of the mandelonitrile solution and the adsorption at 280 nm is monitored for two minutes (blank). Subsequently, the HNL sample (100 µg) is added and, after mixing, the adsorption is monitored for five minutes. The calculation requires a calibration curve that is obtained by measuring the adsorption at 280 nm with increasing amounts of freshly distilled benzaldehyde in water.

In the present invention, the hydroxynitrile lyase can also be used in immobilized form, which has the advantage that it makes higher enzyme loadings possible and facilitates recycling. In that case, the volume of the aqueous salt solution can further be reduced (to ratios of organic solvent and aqueous salt solution up to 50:1) because in that case water is absorbed by the carrier, which is sufficient for the enzyme to be catalytically active and which is also in favor of the enantioselectivity of the reaction. Suitable carrier materials for non-covalent immobilization of HNLs include cellulose and celite.

The process used in the present invention is typically performed by dissolving the aldehyde or ketone in the water-immiscible organic solvent which contains the hydrogen cyanide. A solution of hydroxynitrile lyase in the aqueous salt solution is then added to the organic phase. The resulting mixture is stirred until a conversion of at least 80%, preferably at least 90%, more preferably at least 95%, and even more preferably of at least 97.5% has been reached. The rate of conversion can be controlled by common techniques, such as e.g. GC or HPLC.

The invention is illustrated in the following examples.

### Examples

### Example 1: Hydrocyanation of 4-hydroxybenzaldehyde catalyzed by R-HNL

Experiment **1A** (reference example at low buffer concentration of 0.05 mol/L): 4-Hydroxybenzaldehyde (0.061 g, 0.5 mmol) was dissolved in 0.5 mL di-*iso*-propylether containing 3.2M HCN. The mixture was cooled to 0°C and 0.1 mL 50 mM potassium citrate buffer pH 5 containing 147 U (*R*)-HNL (corresponding to 294 U per mmol aldehyde) was added and the mixture was stirred at 0°C. Samples were periodically taken and analyzed by chiral HPLC.

Product yield and enantiomeric excess (ee) measured by chiral HPLC at different time points are summarized in the following **Table 1.**

**Table 1.**

| **Time, h** | **Cyanohydrin, mM** | | **HPLC yield (*R*+*S* Cyanohydrin)** | **ee (overall)** |
|---|---|---|---|---|
| | ***S*** | ***R*** | | |
| 2 | 4.1 | 356 | 36.0% | 97.7% (*R*) |
| 3.5 | 6.5 | 365 | 37.1% | 96.5% (*R*) |
| 5 | 9.5 | 373 | 38.3% | 95.0% (*R*) |
| 23 | 33.0 | 391 | 42.4% | 84.5% (*R*) |
| 45 | 59.1 | 428 | 48.7% | 75.7% (*R*) |

Experiment **1B** (at high buffer concentration of 1 mol/L): 4-Hydroxybenzaldehyde (0.488 g, 4 mmol) was dissolved in 4 mL di-*iso*-propylether containing 3.2M HCN. The mixture was cooled to 0°C and 0.8 mL 1M potassium citrate buffer pH 5 containing 1180 U (*R*)-HNL (corresponding to 295 U/mmol aldehyde) was added and the mixture was stirred at 0°C. Samples were periodically taken and analyzed by chiral HPLC.

Product yield and enantiomeric excess (ee) measured by chiral HPLC at different time points are summarized in the following **Table 2.**

**Table 2.**

| **Time, h** | **Cyanohydrin, mM** | | **HPLC yield (*R*+*S* Cyanohydrin)** | **ee (overall)** |
|---|---|---|---|---|
| | ***S*** | ***R*** | | |
| 1 | 0.9 | 485 | 48.6% | 99.6% (*R*) |
| 2 | 1.0 | 582 | 58.3% | 99.6% (*R*) |
| 3 | 1.4 | 607 | 60.8% | 99.6% (*R*) |
| 18 | 3.6 | 760 | 76.4% | 99.1% (*R*) |
| 42 | 6.0 | 781 | 78.7% | 98.5% (*R*) |
| 66 | 8.2 | 804 | 81.2% | 98.0% (*R*) |

In Experiment **1B**, wherein the aqueous solution had a high salt concentration of 1M, the enantiomeric excess as well as the conversion at any time point investigated was higher than in the comparative process applied in Experiment **1A,** wherein the aqueous solution had a low salt concentration of 50mM. It is thus demonstrated that carrying out the hydrocyanation process using a highly concentrated aqueous salt solution is beneficial in terms of improving the ee of the reaction.

### Example 2: Hydrocyanation of 4-hydroxybenzaldehyde catalyzed by S-HNL in various salt solutions

4-Hydroxybenzaldehyde (0.066 g, 0.54 mmol) was dissolved in 0.5 mL di-*iso*-propylether containing 3.2M HCN. The mixture was cooled to 0°C and
- Experiment **2A:** 0.2 mL 50mM potassium citrate buffer pH 5 containing 24 U (*S*)-HNL (corresponding to 44 U per mmol aldehyde) was added (reference example);
- Experiment **2B:** 0.2 mL 1M potassium citrate buffer pH 5 containing 24 U (*S*)-HNL (corresponding to 44 U per mmol aldehyde) was added;
- Experiment **2C:** 0.2 mL 0.95M sodium sulfate, 50 mM potassium citrate buffer, pH 5 containing 24 U (*S*)-HNL (corresponding to 44 U per mmol aldehyde) was added;
   and the mixture was stirred at 0°C. Samples were periodically taken and analyzed by chiral HPLC. Product yield and enantiomeric excess (ee) measured by chiral HPLC at different time points are summarized in the following **Table 3.**

**Table 3.**

| **2A:** 50 mM citrate buffer, pH 5 | | | **2B:** 1M citrate buffer pH 5 | | |
|---|---|---|---|---|---|
| **Time, h** | **Conversion** | **ee** | **Time, h** | **Conversion** | **ee** |
| **1** | 6.0% | 84.5% (*S*) | **1** | 10.3% | 92.4% (*S*) |
| **4** | 38.1% | 89.4% (*S*) | **4** | 30.9% | 95.4% (*S*) |
| **28** | 75.5% | 94.8% (*S*) | **28** | 75.1% | 94.8% (*S*) |
| **52** | 77.6% | 86.1% (*S*) | **52** | 83.1% | 94.1% (*S*) |
| **68** | 80.3% | 86.0% (*S*) | **68** | 84.7% | 91.4% (*S*) |
| **92** | 83.5% | 84.5% (*S*) | **92** | 86.9% | 92.8% (*S*) |

| **2C:** 50 mM citrate buffer/0.95M Na₂SO₄ pH 5 | | | | | |
|---|---|---|---|---|---|
| **Time, h** | **Conversion** | **ee** | | | |
| **1** | 16.0% | 94.9% (*S*) | | | |
| **2** | 18.5% | 95.3% (*S*) | | | |
| **25** | 74.5% | 95.0% (*S*) | | | |
| **40** | 82.6% | 95.1% (*S*) | | | |

The example shows a quick deterioration of the ee after 75% conversion when carrying out the reaction using an aqueous salt solution at a low salt concentration of 50mM (Experiment **2A),** while the ee was better maintained when carrying out the reaction at a higher salt concentration of 1M (Experiments **2B** and **2C).**

### Example 3: Hydrocyanation of 4-hydroxybenzaldehyde catalyzed by R-HNL at higher enzyme loading

With the results of Experiment **2C** at hand, the enantioselectivity of the reaction could be further improved by increasing the enzyme loading. For this purpose, 4-hydroxybenzaldehyde (0.066 g, 0.54 mmol) was dissolved in 0.5 mL di-*iso*-propylether containing 3.2M HCN. The mixture was cooled to 0°C and 0.2 mL 0.95M sodium sulfate, 50mM potassium citrate buffer pH 5 containing 112 U (R)-HNL (corresponding to 207 U per mmol aldehyde) was added and the mixture was stirred at 0°C (Experiment **3A).** Samples were periodically taken and analyzed by chiral HPLC. Product yield and enantiomeric excess (ee) measured by chiral HPLC at different time points are summarized in the following **Table 4.**

**Table 4.**

| **3A:** 50 mM citrate / 0.95M Na₂SO₄ pH 5 | | |
|---|---|---|
| **Time, h** | **Conversion** | **ee** |
| **1** | 66.2% | 99.7% |
| **2** | 74.1% | 99.8% |
| **24** | 82.2% | 99.0% |
| **48** | 83.2% | 98.1% |

Thus, by increasing the enzyme loading compared to Experiment **2C,** the ee of the reaction could be further improved to 98.1% at a conversion of 83.2% after 48 hours.

### Example 4: Enzymatic hydrocyanation of frambinone, catalyzed by (S)-HNL

Frambinone (0.0205 g, 0.125 mmol) was dissolved in 0.5 mL di-*iso*-propylether containing 3.2M HCN. The mixture was cooled to 0°C and 0.2 mL of a solution of 8 U (S)-HNL in
- Experiment **4A:** 0.2 mL of a solution of 8 U (S)-HNL (corresponding to 64 U per mmol ketone) in 50 mM potassium citrate buffer pH 5 was added (reference example);
- Experiment **4B:** 0.2 mL of a solution of 8 U (S)-HNL (corresponding to 64 U per mmol ketone) in 1M potassium citrate buffer pH 5 was added;
and the mixture was stirred at 0°C. Samples were periodically taken and analyzed by chiral HPLC. Product yield and enantiomeric excess (ee) measured by chiral HPLC at different time points are summarized in the following **Table 5.**

**Table 5.**

| 4A: Reaction in 50mM citrate buffer, pH 5 | | | **4B:** Reaction in 1M citrate buffer, pH 5 | | |
|---|---|---|---|---|---|
| **Time, h** | **Conversion** | **ee** | **Time, h** | **Conversion** | **ee** |
| **1** | 26.8% | 84.6% (*S*) | **1** | 13.4% | 77.6% (*S*) |
| **2** | 44.9% | 81.2% (*S*) | **2** | 27.5% | 81.5% (*S*) |
| **3** | 58.4% | 80.6% (*S*) | **3** | 41.8% | 80.2% (*S*) |
| **4** | 70.5% | 80.3% (*S*) | **7** | 65.4% | 81.7% (*S*) |
| **24** | 96.6% | 76.6% (*S*) | **24** | 95.3% | 80.0% (*S*) |
| **48** | 97.3% | 72.4% (*S*) | **48** | 96.8% | 78.3% (*S*) |

The results show that when carrying out the reaction at a high salt concentration of 1M (Experiment **4B**) compared to a low salt concertation of 50mM (Experiment **4A**), a better enantioselectivity is achieved.

### Example 5: Enzymatic hydrocyanation of furfural

Furfural (0.048 g, 0.5 mmol) was dissolved in 0.5 mL di-*iso*-propylether containing 3.2M HCN. At room temperature,
- Experiment **5A:** 0.2 mL of a solution of 0.8 U (S)-HNL (corresponding to 1.6 U per mmol aldehyde) in 50 mM potassium citrate buffer pH 5 was added (reference example);
- Experiment **5B:** 0.2 mL of a solution of 0.8 U (S)-HNL (corresponding to 1.6 U per mmol aldehyde) in 50 mM potassium citrate buffer and 0.95 sodium sulfate pH 5 was added;
   and the mixture was stirred at room temperature. Samples were periodically taken and analyzed by chiral HPLC. Product yield and enantiomeric excess (ee) measured by chiral HPLC at different time points are summarized in the following **Table 6.**

**Table 6.**

| Experiment **5A** 50mM potassium citrate, pH 5 | | | Experiment **5B** 50mM potassium citrate, 0.95M sodium sulfate, pH 5 | | |
|---|---|---|---|---|---|
| **Time, h** | **Conversion, %** | **ee, %** | **Time, h** | **Conversion, %** | **ee, %** |
| **0.25** | 88.5 | 51.8 | **0.25** | 92.9 | 89.7 |
| **0.5** | 95.6 | 38.4 | **0.5** | 97.4 | 82.3 |
| **1.5** | 99.0 | 26.1 | **1.5** | 98.7 | 77.3 |

This example shows a significant effect on the enantiomeric excess of the cyanohydrin formed in the initial fast phase of the process , as well as on the stability of the enantiomeric excess in the later phase of the process if the process is carried out using an aqueous salt solution at a high concentration of 1M (Experiment **5B**) compared to an aqueous salt solution at a low concentration of 50mM (Experiment **5A**).

## Claims

1. Use of an aqueous salt solution having a concentration of at least 0.8 mol/L for improving the enantioselectivity in a process for the preparation of optically active cyanohydrins from aldehydes and ketones with hydrogen cyanide in the presence of hydroxynitrile lyase in a biphasic solvent system further comprising an organic solvent.

2. Use according to claim 1, wherein the aqueous salt solution comprises a buffer salt solution or a mixture of a buffer salt solution and a non-buffer salt solution.

3. Use according to claim 2, wherein the buffer is a glutamate, phosphate, acetate or citrate buffer.

4. Use according to any one of the preceding claims, wherein the aqueous salt solution comprises a buffer and an alkali or earth alkali salt, preferably an alkali or earth alkali sulfate.

5. Use according to any one of the preceding claims, wherein the aqueous salt solution comprises a mixture of a citrate buffer and sodium sulfate.

6. Use according to any one pf the preceding claims, wherein the concentration of the aqueous salt solution is at least 1 mol/L, preferably at least 1.2 mol/L, more preferably at least 1.4 mol/L.

7. Use according to any one of the preceding claims, wherein hydroxynitrile lyase is used in an amount in the range from 1 to 1000 U per mmol of the aldehyde or ketone.

8. Use according to any one of the preceding claims, wherein the pH of the aqueous salt solution is in the range from 3 to 7.

9. Use according to any one of the preceding claims, wherein the organic solvent is an aliphatic ether or a mixture of aliphatic ethers, preferably di-*iso*-propylether and methyl-*tert-*butylether.

10. Use according to any one of the preceding claims, wherein the process is carried out at a temperature ranging from 0°C to 30°C.

11. Use according to any one of preceding claims, wherein the aldehydes and ketones are compounds of the following formula (I) 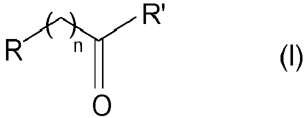 wherein in formula (I)
n = 0, 1 or 2,
R = aryl, heteroaryl or alkyl
R' = H or C1-C4 alkyl,
wherein
R = aryl means a moiety of the following formula (II) 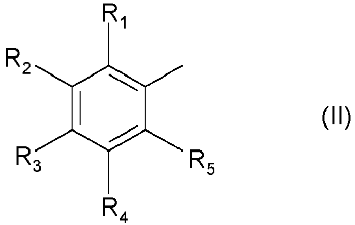 wherein
R₁-R₅ are independently selected from -H, -F, -Cl, -Br, -I, -OH, -O(C1-C4 alkyl), -O-benzyl, -OCO(C1-C4 alkyl), -OCO-benzyl, -NH₂, - NH(C1-C4 alkyl), -NH-benzyl, -NHCO(C1-C4 alkyl), -N-HCO-benzyl, - NO₂, -COOH, -COO(C1-C4 alkyl), -COO-benzyl, wherein preferably at least one of R₁-R₅ is -OH;
R = heteroaryl means a moiety selected from the group consisting of 2-pyrrolyl, 3-pyrrolyl, 2-furyl, 3-furyl, 2-thienyl, 3-thienyl, 2-pyridyl, 3-pyridyl; R = alkyl means C1-C4 alkyl, optionally substituted with -OH, -O(C1-C4 alkyl), -O-benzyl, -OCO(C1-C4 alkyl), -OCO-benzyl, -NH₂, -NH(C1-C4 alkyl), -NH-benzyl, -NHCO(C1-C4 alkyl), -N-HCO-benzyl, -NO₂, -COOH, -COO(C1-C4 alkyl), -COO-benzyl.

12. Use according to claim 11, wherein in the compound of the formula (I) n = 0, 1 or 2, R = aryl or heteroaryl, and R' = H or C1-C4 alkyl.

13. Use according to claim 11 or 12, wherein in the compound of the formula (I) n = 0, R = aryl or heteroaryl, and R' = H

14. Use according to any one of claims 11 to 13, wherein the compound of formula (I) is selected from hydroxy- and di-hydroxy-substituted benzaldehyde analogues.

15. Use according to any of the preceding items, wherein the hydroxynitrile lyase is immobilized hydroxynitrile lyase.
